# EUROPÄISCHE PATENTSCHRIFT

(11) **EP 2 990 787 B1**
(45) Veröffentlichungstag und Bekanntmachung des Hinweises auf die Patenterteilung: **17.05.2017**
(21) Anmeldenummer: 15180931.6
(22) Anmeldetag: 13.08.2015
(51) Int. Cl.: G01N 27/447, G01N 15/10, G01N 33/49, G01N 33/50

(54) **VERFAHREN ZUR MESSUNG DER THROMBOZYTENFUNKTION**
METHOD FOR MEASURING THROMBOCYTE FUNCTION
PROCEDE DE MESURE DE LA FONCTION DES THROMBOCYTES

(30) Priorität: 27.08.2014 DE 102014112270
(43) Veröffentlichungstag der Anmeldung: 02.03.2016
(73) Patentinhaber: Leibniz - Institut für Analytische Wissenschaften - ISAS - e.V., 44139 Dortmund (DE)
(72) Erfinder: West, Jonathan, Southampton, Hampshire SO14 6HL (GB); Janasek, Dr., Dirk, 44139 Dortmund (DE); Sickmann, Prof. Dr., Albert, 44143 Dortmund (DE)
(74) Vertreter: Meinke, Jochen

(56) Entgegenhaltungen:
- EP-A1- 1 742 057
- US-A1- 2014 200 240
- WARD ET AL: "A sensitive microelectrophoretic method for measuring the anti-platelet activity of horse anti-human lymphocyte serum", JOURNAL OF IMMUNOLOGICAL METHODS, ELSEVIER SCIENCE PUBLISHERS B.V.,AMSTERDAM, NL, Bd. 6, Nr. 1-2, Dezember 1974 (1974-12), Seiten 121-128, XP025446422, ISSN: 0022-1759, DOI: 10.1016/0022-1759(74)90097-0 [gefunden am 1974-12-01]

## Beschreibung

Die Erfindung betrifft ein Verfahren zur Messung der Thrombozytenfunktion.

Es besteht ein Bedarf an Thrombozyten-Funktionstests im human- und veterinärmedizinischen Bereich, welche Störungen der Thrombozyten (Blutplättchen) und der Hämostase diagnostizieren und Antikoagulationstherapien überwachen können, dabei vorhersageverlässlicher und kosteneffektiver sind und außerhalb klinischer Laboratorien eingesetzt werden können. Die gegenwärtig verfügbaren Thrombozyten-Funktionstests messen die Reaktionsantwort einer Thrombozyten-Population (mehrere Millionen Thrombozyten) auf Agonisten unter statischen Bedingungen. Das reflektiert nicht in-vivo-Bedingungen, wo parakrine Signale feinverteilt sind, und betrachtet auch nicht die inhärenten Unterschiede in der funktionalen Kapazität (z.B. Sensitivität) zwischen unterschiedlichen Thrombozyten, insbesondere mit Blick auf hypersensitive Thrombozyten.

Die Licht-Transmissions-Aggregometrie ist der sogenannte "Gold-Standard" bei Thrombozyten-Funktionstests, wohingegen die Impedanz-Aggregometrie wegen verschiedener Faktoren, welche die Präzision beeinflussen, nicht umfangreich genutzt wird. Gegenwärtig analysieren alle Thrombozyten-Tests Antworten einer Thrombozyten-Population auf Agonisten (Stimulanzien) geben aber keine Information auf der Ebene der einzelnen Thrombozyten.

Aus WO 2007/008064 A2 ist ein Free-Flow-Elektrophorese-System mit einer mikrofluidischen Kammer bekannt, welches, wie bei Free-Flow-Elektrophorese-Systemen hinlänglich bekannt ist, dazu dient, Partikel aufgrund ihrer elektrischen Ladungen voneinander zu trennen und zu analysieren und die so getrennten Partikel ggf. in einer nachgeschalteten Detektionseinheit weiter zu beeinflussen.

Aus WO 2013/013228 A1 ist ein Verfahren bekannt, das in erster Linie zur Untersuchung von Blutproben von Patienten dient, welchen zuvor ein Medikament verabreicht wurde, um die Wirksamkeit des Medikamentes zu überprüfen. Alternativ kann auch vorgesehen sein, ein derartiges Medikament bzw. eine entsprechende Substanz, ohne diese zuvor den Patienten zu verabreichen, direkt zur Blutprobe zuzugeben und danach die entsprechenden Tests durchzuführen. Anschließend, also nach der Einwirkung des Medikamentes bzw. einer entsprechenden Substanz auf die Blutprobe wird die Probe in ein elektrisches Feld eingeleitet und es wird eine Unterscheidung von aktivierten und nicht aktivierten Thrombozyten anhand der unterschiedlichen Auslenkung im elektrischen Feld durchgeführt.

Aus US 7 138 269 B2 sind mikrofluidische Systeme zur Charakterisierung von Partikeln und Blutzellen bekannt, bei denen ein elektrisches Feld Verwendung finden kann, um unterschiedlich geladene Partikel durch unterschiedliche Bewegung im mikrofluidischen System zu kennzeichnen, wobei die einzelnen Partikel individuell untersucht werden können. Dabei kann der zu untersuchenden Lösung mit den Partikeln vor der Untersuchung ein Reagenz zugegeben werden.

Ward et al. (Journal of Immunological Methods 1974, Bd. 6, Nr. 1-2, 121-128) offenbart ein Verfahren zur Messung der Thrombozytenfunktion, wobei eine flüssige Thrombozytenlösung in eine Elektrophoresekammer, an die ein sich in Eintrittsrichtung der Thrombozytenlösung gerichtetes elektrisches Feld angelegt wird, geleitet und unmittelbar vor der Einwirkung des elektrischen Feldes in Kontakt mit einem Stimulanz (*horse serum*) gebracht und die Geschwindigkeit jedes einzelnen Thrombozyten im elektrischen Feld beobachtet und ausgewertet wird. Eine geringere Mobilität legt dabei eine stärkere Wirkung des *horse serums* auf die Thrombozyten nahe. Aufgabe der Erfindung ist es, eine Lösung zur Verfügung zu stellen, mittels welcher die Empfindlichkeit einzelner Thrombozyten im Hochdurchsatz mit möglichst geringem apparativen Aufwand gemessen werden kann.

Diese Aufgabe wird bei einem Verfahren der eingangs bezeichneten Art erfindungsgemäß dadurch gelöst, dass eine flüssige Thrombozytenlösung, in welcher die Thrombozyten vereinzelt vorliegen, in eine mikrofluidische Kammer, an die ein sich quer zur Eintrittsrichtung der Thrombozytenlösung gerichtetes elektrisches Feld angelegt wird, geleitet und unmittelbar vor oder während der Einwirkung des elektrischen Feldes in Kontakt mit wenigstens einem Stimulanz gebracht und die Bewegungsbahn der Thrombozyten im elektrischen Feld beobachtet und ausgewertet wird, derart, dass Thrombozyten mit einer in Richtung zum Minuspol des elektrischen Feldes gerichteten Bewegungsbahn als aktivierte Thrombozyten und Thrombozyten mit einer in Richtung zum Pluspol des elektrischen Feldes gerichteten Bewegungsbahn als nicht aktivierte Thrombozyten eingestuft werden.

Mit der Erfindung wird ein Verfahren zur Verfügung gestellt, mit dem es mit geringem apparativen Aufwand im Hochdurchsatz möglich ist, die Empfindlichkeit einzelner Thrombozyten zu messen. Dazu wird eine Thrombozytenlösung, in welcher die Thrombozyten vereinzelt vorliegen, also z.B. eine stark verdünnte Thrombozytenlösung, unmittelbar vor oder während der Einwirkung eines elektrischen Feldes mit einem Stimulanz zusammengeführt, welches auf die Thrombozyten einwirkt. Je nach Wirksamkeit des Stimulanz (des Agonisten) verändert sich in bekannter Weise die Oberfläche des einzelnen Thrombozyten und damit sein elektrischer Ladungszustand. Bekanntlich ist die Oberflächenladung eines nicht aktivierten Thrombozyten negativ und eines aktivierten Thrombozyten weniger negativ bis positiv. Je nach erfolgter oder nicht erfolgter Aktivierung des jeweiligen Thrombozyten durch das Stimulanz (bei Verwendung eines Aktivators als Stimulanz) ändert sich die Bewegungsbahn des einzelnen Thrombozyten im elektrischen Feld, welche mikroskopisch auf einfache Weise beobachtet werden kann. Genauso kann bei Verwendung eines Inhibitors als Stimulanz die Wirksamkeit des Inhibitors auf den jeweiligen Thrombozyten durch Beobachtung der Bewegungsbahn des Thrombozyten überprüft werden. Je nach Verlauf der Bewegungsbahn ist dann eine Einstufung des Thrombozyten auf einfache Weise möglich. Das elektrische Feld in der mikrofluidischen Kammer wird somit nicht zur (Partikel-)Trennung benutzt, sondern es wird die Änderung in den Bewegungsbahnen ausgewertet und es erfolgt eine Vermischung der Partikel (Thrombozyten) unmittelbar vor oder während der Einwirkung des elektrischen Feldes mit einem Stimulanz.

In besonders bevorzugter Ausgestaltung ist vorgesehen, dass die Thrombozytenlösung zunächst in Kontakt mit einem ersten Stimulanz und anschließend stromabwärts in der Kammer in Kontakt mit einem zweiten Stimulanz gebracht wird. Das erste Stimulanz ist dann bevorzugt ein Aktivator und das zweite ein Inhibitor.

In bevorzugter weiterer Ausgestaltung ist vorgesehen, dass die flüssige Thrombozytenlösung in eine mikrofluidische Free-Flow-Elektrophorese-Kammer geleitet wird.

Die Zusammenführung oder Vermischung der Thrombozytenlösung mit dem Stimulanz kann auf unterschiedliche Weise erfolgen.

Nach einer ersten Ausgestaltung ist vorgesehen, dass die flüssige Thrombozytenlösung zunächst mit einer Stimulanzlösung zusammengeführt und anschließend die vermischte Thrombozyten-Stimulanzlösung in die mikrofluidische Kammer geleitet wird. Die Vermischung erfolgt bei dieser Ausgestaltung somit unmittelbar vor der Einleitung in die mikrofluidische Kammer.

Nach einer zweiten Ausgestaltung ist vorgesehen, dass die flüssige Thrombozytenlösung und eine Stimulanzlösung parallel in die mikrofluidische Kammer geleitet werden. Die Vermischung erfolgt dann erst im Wirkungsbereich des elektrischen Feldes.

Schließlich ist alternativ vorgesehen, dass vor Einleitung der Thrombozytenlösung in die mikrofluidische Kammer auf dem Boden der Kammer Stimulanzpartikel immobilisiert werden. Die Thrombozytenlösung und damit die Thrombozyten kommen dann mit den stationär in der Kammer angeordneten Stimulanzpartikeln in Kontakt. Die Immobilisierung auf dem Boden der Kammer ist auch besonders gut für das Inkontaktbringen der Thrombozyten mit einem zweiten Stimulanz geeignet.

Unter einer Stimulanzlösung sind im Sinne der Erfindung nicht nur Lösungen an sich, sondern auch Suspensionen zu verstehen, in denen nicht gelöste Stimulanzpartikel vorliegen.

Als Stimulanzien können grundsätzlich alle zur Thrombozytenbehandlung bekannten und geeigneten verwendet werden, bevorzugt werden Adenosindiphosphat, Kollagen, Thrombin oder Prostaglandin als Aktivator und Acetylsalicylsäure, Convulxin, Clopidogrel, Prasugrel, Ticagrelor, Prostacyclin als Inhibitor verwendet.

Als fluides Trägermedium für die Thrombozyten in der Thrombozytenlösung kann z.B. ein Hepes-Puffer verwendet werden.

Zur Auswertung der Bewegungsbahn der Thrombozyten kann nach einer ersten Ausgestaltung bevorzugt vorgesehen sein, dass die Bewegungsbahn der Thrombozyten in der Kammer mittels bildgebender Verfahren oder optischer Detektionsverfahren abgebildet wird.

Das elektrische Feld in der mikrofluidischen Kammer kann mittels Gleichspannung oder mittels gepulster Gleichspannung erzeugt werden. Die Elektroden zur Erzeugung des elektrischen Feldes können in der mikrofluidischen Kammer angeordnet sein. Es können mikrofabrizierte Elektroden sein oder Drahtelektroden. Die Elektroden bestehen z.B. aus Gold, Platin, Graphit oder anderem leitfähigen Material, welches auf ein transparentes Substrat, wie Glas oder Polymethylmethaacrylat gedruckt sein kann oder sie sind drahtförmig aus diesen Materialien hergestellt.

Die Elektroden sind innerhalb der mikrofluidischen Kammer vom eigentlichen Flusskanal durch eine Grenzzone (z.B. Elektrolytbrücken) getrennt, welche aus einer Polymer-Matrix wie einem Hydrogel oder einem ähnlichen Material gebildet sein kann oder welche durch eine Reihe von Mikrokanälen verkörpert wird.

Die Erfindung ist nachstehend anhand der Zeichnung beispielhaft näher erläutert. Diese zeigt in
- Fig. 1: eine schematische Darstellung der prinzipiellen Oberflächenstruktur von nicht aktivierten und aktivierten Thrombozyten,
- Fig. 2: eine Draufsicht auf eine Free-Flow-Elektrophorese-Kammer mit einer ersten Ausgestaltung der Thrombozytenzuführung,
- Fig. 3: eine Free-Flow-Elektrophorese-Kammer in Draufsicht mit einer zweiten Ausgestaltung der Zuführung zur Lösung und
- Fig. 4: eine Free-Flow-Elektrophorese-Kammer in Seitenansicht mit einer dritten Ausgestaltung der Thrombozytenzuführung.

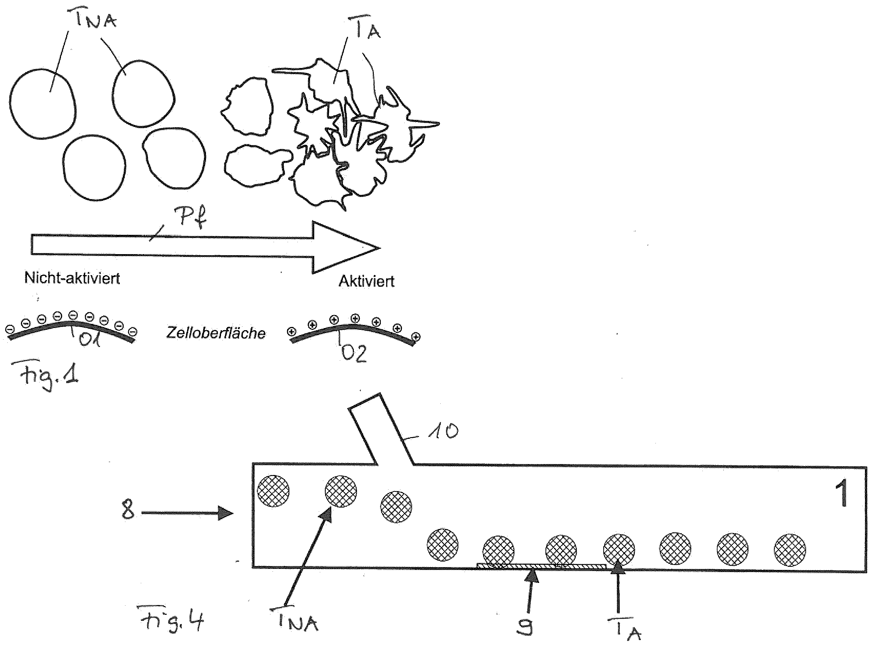

In Fig. 1 sind in weit vergrößertem Maßstab nicht aktivierte rund ausgebildete Thrombozyten mit T_{NA} bezeichnet. Diese nicht aktivierten Thrombozyten T_{NA} weisen an ihrer Oberfläche O1 eine negative Oberflächenladung auf.

Werden diese nicht aktivierten Thrombozyten T_{NA} durch ein Stimulanz bzw. einen Agonisten aktiviert, was durch den Pfeil Pf symbolisch angedeutet ist, nehmen die aktivierten Thrombozyten T_{A} eine andere, nämlich eine unregelmäßige Oberflächenstruktur und Membranbestandteile ordnen sich in der Thrombozytenmembran anders an, so dass die elektrische Ladung an der Oberfläche 02 der aktivierten Thrombozyten T_{A} weniger negativ bis positiv ist. Diese unterschiedliche Oberflächenladungsverteilung von nicht aktivierten Thrombozyten T_{NA} und von aktivierten Thrombozyten T_{A} macht sich das erfindungsgemäße Verfahren zum Nutzen.

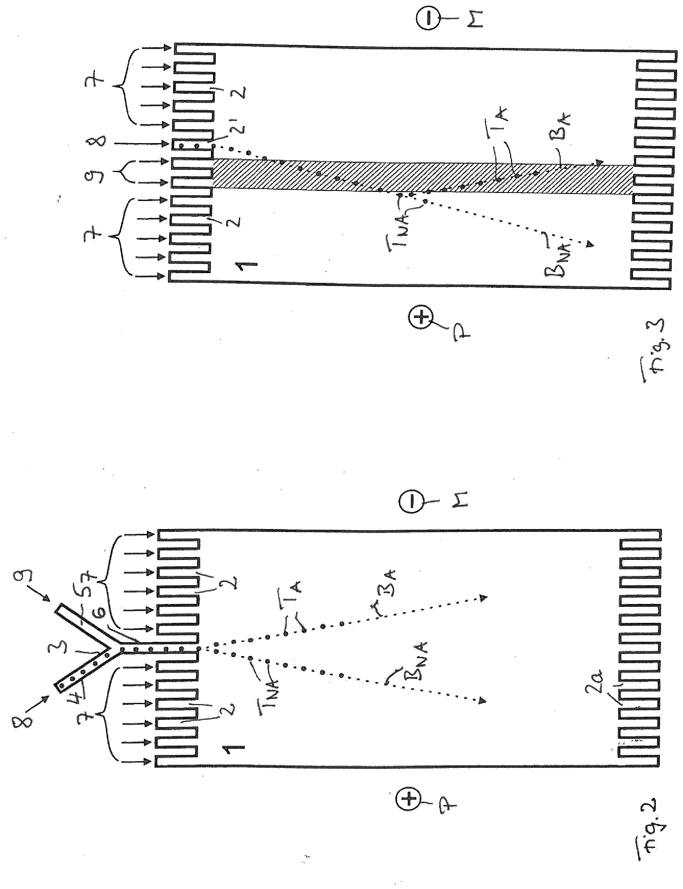

In Fig. 2 ist eine mikrofluidische Kammer in Form einer Free-Flow-Elektrophorese-Kammer 1 dargestellt, an welche quer zur in Längsrichtung der Kammer 1 erstreckten Hauptströmungsrichtung ein elektrisches Feld angelegt wird, welches durch einen Pluspol P und einem Minuspol M angedeutet ist. Es sind entsprechend geeignete Elektroden in den beiderseitigen Randbereichen der Kammer 1 angeordnet oder in die Kammer 1 integriert, wenn die Elektroden aufgedruckte Elektroden sind.

Die Free-Flow-Elektrophorese-Kammer 1 weist eine Mehrzahl paralleler Eintrittskanäle 2 und Austrittskanäle 2a auf. Beim Ausführungsbeispiel gemäß Fig. 2 ist an einem zentralen Eintrittskanal eine Y-förmige Zuleitung 3 angeschlossen, durch deren einen Zulauf 4 eine Thrombozytenlösung 8 mit einzelnen nicht aktivierten Thrombozyten T_{NA} und durch deren anderen Zulauf 5 eine Stimulanzlösung 9 zugeführt wird, welche sich in einem Mischbereich 6 der Zuleitung 3 vermischen und vermischt in die Kammer 1 gelangen. Parallel dazu wird durch die weiteren Kanäle 2 ein Laufpuffer 7 in die Kammer 1 geleitet, so dass die Kammer 1 über ihrer gesamten Breite und Länge durchströmt ist.

Die Thrombozytenlösung 8 besteht neben den vereinzelt in der Lösung enthaltenen, nicht aktivierten Thrombozyten T_{NA} vorzugsweise aus einem Hepes-Puffer, auch der Laufpuffer 7 kann ein Hepes-Puffer, ggf. aber auch eine Stimulanzlösung sein.

Im Mischbereich 6 der Y-förmigen Zuleitung 3 wirkt die Stimulanzlösung 9 auf die Thrombozyten ein, wodurch einige Thrombozyten aktiviert werden und ihre Oberflächenladung sich von negativ nach weniger negativ bis positiv verändert.

Dies führt dazu, dass im Einflussbereich des elektrischen Feldes innerhalb der Kammer 1 sich die Bewegungsbahn B_{NA} der nicht aktivierten Thrombozyten T_{NA} in Richtung des Pluspoles P erstreckt, während sich die Bewegungsbahn B_{A} der aktivierten Thrombozyten T_{A} in Richtung des Minus-Poles M erstreckt.

Erfindungsgemäß wird die jeweilige Bewegungsbahn jedes einzelnen Thrombozyten detektiert, beispielsweise mittels eines Mikroskopes, Thrombozyten mit einer zum Pluspol gerichteten Bewegungsbahn werden als nicht aktivierte Thrombozyten T_{NA} und Thrombozyten mit einer zum Minuspol gerichteten Bewegungsbahn B_{A} als aktivierte Thrombozyten T_{A} eingestuft und ausgewertet (also qualifiziert).

Beim Ausführungsbeispiel nach Fig. 3 ist gegenüber dem Ausführungsbeispiel nach Fig. 2 die Zuleitung der Thrombozytenlösung 8 und der Stimulanzlösung 9 in die Kammer 1 anders, ansonsten unterscheidet sich das Ausführungsbeispiel nicht gegenüber dem Ausführungsbeispiel nach Fig. 2. Die Stimulanzlösung 9 wird beim Ausführungsbeispiel nach Fig. 3 zentral in die Einlasskanäle 2 eingeleitet und fließt im Wesentlichen entlang des schraffierten Bereiches durch die Kammer 1. Die Thrombozytenlösung 8 wird durch einen benachbart dazu angeordneten Kanal 2' eingeleitet. Durch die Wirkung des elektrischen Feldes in der Kammer 1 werden die zunächst nicht aktivierten Thrombozyten T_{NA} in Richtung des Pluspoles ausgelenkt und gelangen dadurch in Kontakt mit der Stimulanzlösung 9. Diejenigen Thrombozyten, die nicht aktiviert bleiben, also die Thrombozyten T_{NA,} bewegen sich entlang einer mit B_{NA} dargestellten Bewegungsbahn in Richtung des Pluspoles P. Durch das Inkontaktkommen mit der Stimulanzlösung dagegen aktivierte Thrombozyten T_{A} verändern ihre Bewegungsbahn, ihre Bewegungsbahn B_{A} ist letztendlich in Richtung des Minuspoles M gerichtet. Durch die Beobachtung der Bewegungsbahn B_{NA}, B_{A} der einzelnen Thrombozyten lassen sich diese somit auch bei diesem Ausführungsbeispiel einstufen, Thrombozyten mit einer letztendlich zum Pluspol P gerichteten Bewegungsbahn B_{NA} sind nicht aktivierte Thrombozyten T_{NA} und Thrombozyten mit einer letztendlich zum Minuspol gerichteten Bewegungsbahn B_{A} sind aktivierte Thrombozyten T_{A}.

In Fig. 4 ist eine weitere Ausgestaltung dargestellt, die Free-Flow-ElektrophoreseKammer 1 ist gegenüber der Darstellung nach Fig. 2 und 3 um 90° gedreht und von der Seite dargestellt. Bei dieser Ausgestaltung wird keine Stimulanzlösung in die Kammer eingeleitet, sondern Stimulanzpartikel sind auf der Bodenfläche der Kammer 1 immobilisiert.

Die Thrombozytenlösung 8 wird durch die Einlasskanäle 2 zugeführt, genauso wie ein Trennpuffer, welches in Fig. 4 nicht dargestellt ist. Zusätzlich wird durch einen oberseitigen Einlass 10 ein zusätzliches Puffer zur dynamischen Fokussierung in die Kammer 1 eingeleitet.

Thrombozyten, die aktiviert werden, sind wiederum mit T_{A} dargestellt, ihre Bewegungsbahn wird dann quer zur Zeichenebene der Fig. 4 in Richtung des Minuspols abgelenkt. Die Auswertung der Bewegungsbahnen der einzelnen Thrombozyten in der Kammer 1 erfolgt wie vorbeschrieben.

Natürlich ist die Erfindung nicht auf die dargestellten Ausführungsbeispiele beschränkt. Weitere Ausgestaltungen sind möglich, ohne den Grundgedanken zu verlassen. So kann die Thrombozytenlösung zunächst in Kontakt mit einem ersten Stimulanz und anschließend stromabwärts in der Kammer in Kontakt mit einem zweiten Stimulanz gebracht werden. Das erste Stimulanz ist dann z.B. ein Aktivator und kann mit den vereinzelten Thrombozyten in der in den Figuren 2 bis 4 beschriebenen Weise in Kontakt gebracht werden. Das zweite Stimulanz ist dann z.B. ein Inhibitor, der vorzugsweise stromabwärts vom Eintritt in die Kammer 1 auf dem Boden der Kammer 1 immobilisiert ist oder stromabwärts der Kammer 1 in die Kammer 1 eingeleitet wird. Die Bewegungsbahn der Thrombozyten im Eingangsbereich der Kammer 1 spiegelt dann die Wirksamkeit des Aktivators auf die Thrombozyten und die Bewegungsbahn der Thrombozyten stromabwärts die Wirksamkeit des Inhibitors auf die Thrombozyten wieder.

### Bezugszeichenliste:

- 1: Kammer
- 2, 2': Eintrittskanäle
- 2a: Austrittskanäle
- 3: Y-förmige Zuleitung
- 4: Zulauf
- 5: Zulauf
- 6: Mischbereich
- 7: Laufpuffer
- 8: Thrombozytenlösung
- 9: Stimulanzlösung
- 10: Einlass

- T_{NA}: nicht aktivierte Thrombozyten
- T_{A}: aktivierte Thrombozyten
- Pf: Pfeil
- M: Minuspol
- P: Pluspol
- B_{NA}: Bewegungsbahn nicht aktivierter Thrombozyten
- B_{A}: Bewegungsbahn aktivierter Thrombozyten
- O1: Oberfläche eines nicht aktivierten Thrombozyten
- O2: Oberfläche eines aktivierten Thrombozyten

## Patentansprüche

1. Verfahren zur Messung der Thrombozytenfunktion,
**dadurch gekennzeichnet,**
**dass** eine flüssige Thrombozytenlösung, in welcher die Thrombozyten vereinzelt vorliegen, in eine mikrofluidische Kammer, an die ein sich quer zur Eintrittsrichtung der Thrombozytenlösung gerichtetes elektrisches Feld angelegt wird, geleitet und unmittelbar vor oder während der Einwirkung des elektrischen Feldes in Kontakt mit wenigstens einem Stimulanz gebracht und die Bewegungsbahn jedes einzelnen Thrombozyten im elektrischen Feld beobachtet und ausgewertet wird, derart, dass ein Thrombozyt mit einer in Richtung zum Minuspol des elektrischen Feldes gerichteten Bewegungsbahn als aktivierter Thrombozyt und ein Thrombozyt mit einer in Richtung zum Pluspol des elektrischen Feldes gerichteten Bewegungsbahn als nicht aktivierter Thrombozyt eingestuft wird.

2. Verfahren nach Anspruch 1,
**dadurch gekennzeichnet,**
**dass** die Thrombozytenlösung zunächst in Kontakt mit einem ersten Stimulanz und anschließend stromabwärts in der Kammer in Kontakt mit einem zweiten Stimulanz gebracht wird.

3. Verfahren nach Anspruch 1 oder 2,
**dadurch gekennzeichnet,**
**dass** die flüssige Thrombozytenlösung in eine mikrofluidische Free-Flow-Elektrophorese-Kammer geleitet wird.

4. Verfahren nach Anspruch 1, 2 oder 3,
**dadurch gekennzeichnet,**
**dass** die flüssige Thrombozytenlösung zunächst mit einer Stimulanzlösung zusammengeführt und anschließend die vermischte Thrombozyten-Stimulanzlösung in die mikrofluidische Kammer geleitet wird.

5. Verfahren nach Anspruch 1, 2 oder 3,
**dadurch gekennzeichnet,**
**dass** die flüssige Thrombozytenlösung und eine Stimulanzlösung parallel in die mikrofluidische Kammer geleitet werden.

6. Verfahren nach einem der Ansprüche 1 bis 5,
**dadurch gekennzeichnet,**
**dass** vor Einleitung der Thrombozytenlösung in die mikrofluidische Kammer auf dem Boden der Kammer Stimulanzpartikel immobilisiert werden.

7. Verfahren nach einem der Ansprüche 1 bis 6,
**dadurch gekennzeichnet,**
**dass** als Stimulanz Adenosindiphosphat, Kollagen, Thrombin oder Prostaglandin verwendet werden.

8. Verfahren nach einem der Ansprüche 1 bis 6,
**dadurch gekennzeichnet,**
**dass** als Stimulanz Acetylsalicylsäure, Convulxin, Clopidogrel, Prasugrel, Ticagrelor, Prostacyclin verwendet werden.

9. Verfahren nach einem der Ansprüche 1 bis 6,
**dadurch gekennzeichnet,**
**dass** die Thrombozytenlösung zunächst in Kontakt mit einem ersten Stimulanz und anschließend stromabwärts in der Kammer in Kontakt mit einem zweiten Stimulanz gebracht wird, wobei als erste Stimulanz ein Aktivator nach Anspruch 7 und als zweite Stimulanz ein Inhibitor nach Anspruch 8 verwendet werden.

10. Verfahren nach einem der Ansprüche 1 bis 9,
**dadurch gekennzeichnet,**
**dass** die Bewegungsbahn der Thrombozyten in der Kammer mittels bildgebender Verfahren oder optischer Detektionsverfahren abgebildet wird.

## Claims

1. Method for measurement of thrombocyte function,
**characterized in that**
a liquid thrombocyte solution comprising thrombocytes in isolated form is passed into a microfluidic chamber to which an electrical field directed transverse to the entry direction of the thrombocyte solution is applied, the thrombocyte solution is brought into contact with at least one stimulant directly before or during action of the electrical field and the movement path of each individual thrombocyte in the electrical field is observed and evaluated, so that a thrombocyte having a movement path directed towards the minus pole of the electrical field is classified as an activated thrombocyte and a thrombocyte having a movement path directed towards the plus pole of the electrical field is classified as a non-activated thrombocyte.

2. Method according to claim 1,
**characterized in that**
the thrombocyte solution is first brought into contact with a first stimulant, and subsequently, downstream, is brought into contact with a second stimulant in the chamber.

3. Method according to claim 1 or 2,
**characterized in that**
the liquid thrombocyte solution is passed into a microfluidic free-flow electrophoresis chamber.

4. Method according to claim 1, 2 or 3,
**characterized in that**
the liquid thrombocyte solution is first brought together with a stimulant solution, and subsequently the mixed thrombocyte-stimulant solution is passed into the microfluidic chamber.

5. Method according to claim 1, 2 or 3,
**characterized in that**
the liquid thrombocyte solution and a stimulant solution are passed into the microfluidic chamber in parallel.

6. Method according to any of claims 1 to 5,
**characterized in that**
before introduction of the thrombocyte solution into the microfluidic chamber, stimulant particles are immobilized on the bottom of the chamber.

7. Method according to any of claims 1 to 6,
**characterized in that**
adenosine diphosphate, collagen, thrombin or prostaglandin are used as stimulant.

8. Method according to any of claims 1 to 6,
**characterized in that**
acetylsalicylic acid, convulxin, clopidogrel, prasugrel, ticagrelor, prostacyclin are used as stimulant.

9. Method according to any of claims 1 to 6,
**characterized in that**
the thrombocyte solution is first brought into contact with a first stimulant, and subsequently, downstream, is brought into contact with a second stimulant in the chamber, wherein an activator according to claim 7 is used as first stimulant and an inhibitor according to claim 8 is used as second stimulant.

10. Method according to any of claims 1 to 9,
**characterized in that**
the movement path of the thrombocytes in the chamber is imaged using imaging methods or optical detection methods.

## Revendications

1. Procédé de mesure de la fonction de thrombocytes,
**caractérisé en ce qu'**une solution liquide de thrombocytes dans laquelle se trouvent isolément les thrombocytes est conduite dans une chambre microfluidique à laquelle est appliqué un champ électrique dirigé transversalement par rapport à la direction d'entrée de la solution de thrombocytes, **en ce que**, immédiatement avant ou pendant l'action du champ électrique, elle est mise en contact avec au moins un stimulant et **en ce que** la trajectoire de déplacement de chaque thrombocyte individuel dans le champ électrique est observée et évaluée, de telle sorte qu'un thrombocyte avec une trajectoire de déplacement dirigée en direction du pôle négatif du champ électrique est classé comme thrombocyte activé et un thrombocyte avec une trajectoire de déplacement dirigée en direction du pôle positif du champ électrique est classé comme thrombocyte non activé.

2. Procédé selon la revendication 1, **caractérisé en ce que** la solution de thrombocytes est d'abord mise en contact avec un premier stimulant puis est mise en contact en aval dans la chambre avec un deuxième stimulant.

3. Procédé selon la revendication 1 ou 2, **caractérisé en ce que** la solution liquide de thrombocytes est conduite dans une chambre microfluidique à électrophorèse libre.

4. Procédé selon la revendication 1, 2 ou 3, **caractérisé en ce que** la solution liquide de thrombocytes est d'abord réunie avec une solution de stimulant puis la solution mélangée de thrombocytes et de stimulant est conduite dans la chambre microfluidique.

5. Procédé selon la revendication 1, 2 ou 3, **caractérisé en ce que** la solution liquide de thrombocytes et une solution de stimulant sont conduites en parallèle dans la chambre microfluidique.

6. Procédé selon l'une des revendications 1 à 5, **caractérisé en ce que**, avant l'introduction de la solution de thrombocytes dans la chambre microfluidique, des particules de stimulant sont immobilisées sur le fond de la chambre.

7. Procédé selon l'une des revendications 1 à 6, **caractérisé en ce que**, comme stimulant, on utilise de l'adénosine-diphosphate, du collagène, de la thrombine ou de la prostaglandine.

8. Procédé selon l'une des revendications 1 à 6, **caractérisé en ce que**, comme stimulant, on utilise de l'acide acétylsalicylique, de la convulxine, du clopidogrel, du prasugrel, du tricagrélor, de la prostacycline.

9. Procédé selon l'une des revendications 1 à 6, **caractérisé en ce que** la solution de thrombocytes est d'abord mise en contact avec un premier stimulant puis est mise en contact en aval dans la chambre avec un deuxième stimulant, un activateur selon la revendication 7 étant utilisé comme premier stimulant et un inhibiteur selon la revendication 8 étant utilisé comme deuxième stimulant.

10. Procédé selon l'une des revendications 1 à 9, **caractérisé en ce que** la trajectoire de déplacement des thrombocytes dans la chambre est représentée au moyen de procédés fournissant des images ou de procédés de détection optiques.
